# EUROPEAN PATENT APPLICATION

(11) **EP 4 382 051 A1**
(43) Date of publication of application: **12.06.2024**
(21) Application number: 22852392.4
(22) Date of filing: 03.08.2022
(51) Int. Cl.: A61B 8/00, G01N 29/24, B06B 1/06, H04R 17/00

(54) **ULTRASONIC RECEIVER AND SENSOR FOR MEASURING ANISOTROPY IN A SAMPLE BY MEANS OF TORSIONAL WAVES, METHOD AND USES THEREOF**

(30) Priority: 04.08.2021 ES 202130760
(71) Applicant: Universidad de Granada, 18071 Granada (ES)
(72) Inventor: RUS CARLBORG, Guillermo, 18071 Granada (ES)
(74) Representative: TRBL Intellectual Property
(86) International application number: PCT/ES2022/070521
(87) International publication number: WO 2023/012396

(57) **Abstract**

The present invention relates to an annular ultrasonic receiver (1) adapted to receive torsional waves coming from a specimen, said receiver comprising: a receiving ring formed by a plurality of ring sections (4), each of which comprises a side in contact with the specimen; supporting means that support the plurality of ring sections (4); a transducer element (6) connected to each of the ring sections (4); and a plurality of connections, adapted to receive electrical signals coming from the specimen and suited for connection thereof to a measuring instrument. The invention further relates to a sensor that includes the receiver (1) and an ultrasonic emitter (3), both housed in a casing (2); as well as the method of using said transductor to measure mechanical parameters such as elasticity and/or anisotropy, for example, in soft tissue.

## Description

### FIELD OF THE INVENTION

The present invention relates to the field of the elastography, and more specifically, ultrasonic elastography and the development of ultrasonic emission and reception devices based on torsional waves for measuring elastic properties.

Its field of application is the non-destructive analysis of materials and, preferably, the use of ultrasonic waves to quantify the elasticity and anisotropy of these materials.

### BACKGROUND OF THE INVENTION

Torsional waves are a distribution of transverse P-waves propagated along an axis (direction of propagation) in which a movement of particles occurs along a circumference centered on said axis. Therefore, the amplitude of a movement of particles in the plane of generation is proportional to the distance to the axis within the diameter of the transducer. Waves of this type are propagated in solid and semi-solid media, such that by analyzing the variations of the speed of sound in said media, structural information of the medium traversed can be obtained.

A transducer is a device adapted to transform one type of energy into a different type of energy. These include electromechanical transducers, which convert electrical energy into mechanical energy in the form of displacements coupled elastically with voltages, in a bidirectional manner.

Specifically, ultrasonic (US) transducers emit and receive ultrasonic waves, which have no ionizing effects. By analyzing the received waves once they have propagated through a specimen of interest, information on changes in consistency or microstructure in said specimen can be obtained. If the specimen is a quasi-compressible material (for example, soft tissue), then the modulus of compressibility (related to longitudinal wave propagation) and the shear modulus are different. This fact leads to the propagation velocity of torsional waves (S-waves) in the specimen being different from longitudinal waves (P-waves).

However, the P-waves predominate and mask the S-waves (which provide information about the shear modulus).

In soft tissues, it is the shear modulus that varies significantly depending on modifications in the microstructure of said tissue. Therefore, torsional wave-based transducers capable of providing high sensitivity to the detection of S-waves are required. Once the S-waves are obtained, the model-based inverse problem can be applied to characterize the mechanical properties of the material, as disclosed for example in reference N. Bochud and G. Rus ["Probabilistic inverse problem to characterize tissue-equivalent material mechanical properties". IEEE transactions on ultrasonics, ferroelectrics, and frequency control, 59(7), 1443-1456, 2012].

However, current commercial elastography-based devices are not capable of measuring anisotropy of the tissue directly. The use of linear or curved transducers involves insonifying the medium under study to obtain an image that is a projection of the surface of the transducer. To obtain a first approximation of the anisotropy of the tissue, it is necessary to make at least a 180° scan, collecting measurements while the transducer rotates about its own axis. It has been proposed to use rotating machines with a high precision of angular displacement wherein the transducer is fixed, standing out as an efficient methodology in *in vitro* or ex *vivo* settings, wherein the study sample can be arranged at will. Very promising experimental results have been obtained, in addition to being able to generate a two-dimensional map with elastic properties, as anticipated in Nguyen et al. [Nguyen, T. D., Jones, R. E., & Boyce, B. L. (2008). "A nonlinear anisotropic viscoelastic model for the tensile behavior of the corneal stroma. Journal of biomechanical engineering," 130(4)]. However, it is very complicated to adapt this configuration to *in vivo* situations given the complex shapes of human and animal anatomy, which is a major limitation of this technology.

Devices emitting ultrasonic torsional waves are known in the prior art. For example, patent ES2602508 anticipates a transducer comprising an electromechanical actuator stimulated by a signal generator that allows it to generate torsional waves with a higher amplitude. This sensor is suited for small organs with cylindrical symmetry (for example, the uterine cervix), wherein torsional waves require low energy input and are medically safe in *in vivo* conditions.

However, the transducer or sensor disclosed in ES2602508 is not suited for measuring anisotropy because in it all the piezoelectric elements are connected to one another; but rather only serves for measuring stiffness. In this document, the receiver is a non-sectioned ring, which measures the average stiffness for multiple orientations; it does not allow discriminating a certain direction. Such a transducer has been successfully applied in Masso et al. ["In vivo Measurement of Cervical Elasticity on Pregnant Women by Torsional Wave Technique: A Preliminary Study". Sensors, 19(15), 3249, 2019] to determine the stiffness of the cervix during pregnancy (it is an *in vivo* study), anticipating modifications of the elastic behavior of the cervix. However, the study of anisotropy of the tissue has not been addressed in said document.

On the other hand, regarding the study of anisotropy, most of the results in the literature have been based on experimental evidence from tensile tests on histological sections following the direction of the fibers. However, this approach is destructive in nature, which is a limitation that prevents its use for *in vivo* testing.

The structural microarchitecture of soft tissue is recently attracting attention from the biomechanical engineering community and is of increasing interest for clinical diagnosis in a broad spectrum of specialties and pathologies, as reported in Rus et al. ["Why Are Viscosity and Nonlinearity Bound to Make an Impact in Clinical Elastographic Diagnosis?" Sensors, 20(8), 2379, 2020].

Therefore, it is necessary to develop new techniques that allow the quantification of the elasticity and anisotropy of a sample under *in vivo* conditions and in a non-invasive manner, through torsional waves. Furthermore, it is necessary for the measurement to correspond to the contribution of shear waves (also known as S-waves), mitigating the contribution of compressional waves, in order to obtain a reliable measure of anisotropy.

Elastic properties and anisotropy give rise to a new class of biomarkers. These biomarkers, which are direct measures of the mechanical properties of tissue, are closely related to the structural microarchitecture of soft tissue and are therefore ideal for diagnostic applications.

### BRIEF DESCRIPTION OF THE INVENTION

As mentioned in the background section, sensor devices capable of measuring the anisotropy of a soft biological tissue specimen (for example, skin) are not currently available. Said study typically uses tensile studies based on histological sections of the specimen in the preferred direction of the fibers of said specimen.

Another technical problem present in the field is the complexity of the characterization of anisotropy in a flat biological tissue, since the estimation of shear wave velocity can only be obtained in ultrasonic elastography by means of one direction in each measurement taken. This is a significant limitation, as it requires being able to freely arrange the sample in various orientations to measure multiple directions, and this is not always possible in a sample (particularly, this approach is not feasible in *in vivo* conditions). Therefore, this restricts the organs and tissue in which conventional ultrasonic elastography can be applied, as these are required to have certain isotropy for transverse wave propagation. Furthermore, multiple measurements are necessary for a complete characterization of the anisotropy.

In this context, the present invention describes a torsional wave emitting and receiving device, which solves the mentioned technical problems and is adapted to provide a safe anisotropy measurement and depositing a minimum amount of energy on the specimen, which makes it a suited technique even in *in vivo* conditions. Specifically, this device (sensor) allows quantifying, in a non-invasive manner, mechanical parameters, including shear modulus, in at least three directions of the specimen, which allows fully characterizing its anisotropy.

In that sense, in a first inventive aspect, the invention relates to an ultrasonic receiver adapted to receive torsional waves coming from a specimen for the characterization of the mechanical parameters (elastic properties, stiffness, etc.) and anisotropy of said specimen. Said receiver comprises:
- A receiving ring formed by a plurality of ring sections, each of which comprises a side in contact with the specimen adapted to receive torsional waves coming from said specimen, in which the mentioned ring sections are separated from one another and are arranged around an axis of rotation of the receiver along a circumference (360°). That is, the imaginary line passing through the contour of all the sections of the ring defines a complete circumference, although there are gaps between the sections.
- A plurality of transducer elements (preferably piezoelectric or capacitive elements, but they could be of another type), each of them acoustically connected to one of the ring sections.
- Supporting means that support the receiving ring.

Ring sectorization allows discriminating the orientation of each ultrasonic measurement obtained, and accordingly allows analyzing anisotropy of the mechanical parameters thereof.

Capacitive micromachined ultrasonic transducers (abbreviated as CMUT) are used in certain embodiments. Transducers must be capable of efficiently converting torsional waves into detectable electrical signals.

In advantageous alternative embodiments of the receiver, the transducer elements are piezoelectric elements and each one further comprises one or more connections suited for receiving electrical signals and for connection thereof to a measuring instrument. Torsional waves received through the specimen are thereby converted into electrical signals by the piezoelectric transducer elements, and said electrical signals are detected in the connections (which act as measurement points or outlet channels) of the receiver to a measuring instrument (for example, said instrument may consist of an analog/digital (A/D) converter for acquiring said signals or an oscilloscope for visualizing in one of the channels thereof the ultrasonic signal once it has been distorted by the specimen). The connections of the receiver and the measuring instrument can be performed directly or after amplification of the electrical signal received in the connections with an amplifier.

In preferred embodiments of the ultrasonic receiver, the supporting means comprise a base. In certain embodiments, the base has an annular geometry and comprises a complete ring (a single, section-free part) manufactured from non-electrically conductive material. Even more preferably, said base will have the same dimensions as the receiving ring. Furthermore, at least the side of the ring sections in contact with the specimen is made of a biocompatible material. This facilitates the use thereof for *in vivo* measurements. The piezoelectric transducer elements are arranged on the annular base on which they are distributed following the contour thereof, arranged between said base and the receiving ring. In these embodiments, the base comprises a plurality of slots for guiding the connections of said receiver.

A second object of the invention relates to an ultrasonic sensor, suited for emitting and receiving torsional waves through a specimen. Said sensor comprises at least:
- One ultrasonic receiver as described above.
- Generation means for generating one or more electrical excitation signals.
- An ultrasonic emitter attached to the receiver, comprising an electromechanical actuator connected to the generation means for generating an excitation signal, wherein said actuator is adapted to induce the rotation of the ring sections of the receiver around an axis of rotation of the electromechanical actuator of the emitter upon receiving one or more electrical excitation signals. Additionally, the emitter (3) comprises a side adapted to be arranged in contact with the specimen, on the surface thereof, preferably on the same plane on which one or more sides of the receiving ring are arranged.
- Acquisition and storage means for acquiring and storing the signals received in the connections of the receiver and of the emitter independently.

The generation means for generating signal (excitation signal) may comprise means hardware/software for conditioning the excitation signal (amplifying it, filtering it, etc.).

In preferred embodiments of the sensor, the axis of rotation of the receiver and the axis of rotation of the electromechanical actuator of the emitter coincide substantially with one another and with the axis of the sensor.

In some advantageous embodiments of the invention, the ultrasonic sensor further comprises a casing which houses the emitter and the receiver, substantially maintaining the alignment between their axes of rotation; wherein said casing further comprises slots for guiding connections of the emitter.

Additionally, other preferred embodiments of the ultrasonic sensor further comprise:
- one or more securing parts of the emitter, manufactured from non-conductive material and delimiting a housing space in which the emitter is inserted and a plurality of slots for guiding connections of the emitter; and
- optionally, alignment means adapted to position the one or more securing parts to align the axes of rotation of the emitter and of the receiver.

In some advantageous embodiments, the ultrasonic sensor further comprises attenuators arranged between the casing and the emitter, and between the casing and the receiver, with said attenuators being adapted to dissipate the mechanical wave which is propagated through the casing.

In other advantageous embodiments of the sensor of the invention, the supporting means comprise a plurality of support platforms for the piezoelectric transducer elements, with at least two side securing posts emerging from each of said platforms; and wherein each post further comprises a plurality of slots for guiding the connections of the receiver.

In preferred embodiments, the ultrasonic sensor is characterized in that the electrodes of each piezoelectric transducer element have connections independent from one another, suited for being measured by a measuring instrument (for example, an oscilloscope) and are connected to the side internal of the receiving ring with silver conductive resin. Advantageously, this allows quantifying the mechanical parameter in several orientations of the specimen simultaneously and independently, since each piezoelectric transducer element is only connected to its own connections, and the anisotropy exhibited by said mechanical parameter can thereby be derived.

In certain preferred embodiments of the invention, the ultrasonic sensor further comprises a Faraday cage covering the electromechanical actuator of the emitter and isolating the connections of the emitter and receiver from one another to eliminate electrical noise from the emitter and receiver resonance. Advantageously, the connections of the emitter and receiver are separated and isolated from one another to prevent electrical noise. That is a result of the use of the securing part(s) separating the casing and the ultrasonic emitter, while at the same time providing stiffness and stability to the assembly of the sensor.

In certain preferred embodiments, the sensor comprises two ring sections and two piezoelectric transducer elements oriented respectively with respect to the axis of the sensor at 0° and 90°. Optionally, the sensor may comprise a third ring section and a third piezoelectric transducer element oriented with respect to the axis of the sensor at 45° or 225°.

Moreover, for a correct measurement, it is necessary to have control of the insonified volume with the torsional waves. Advantageously, the sensor described above allows a penetration depth in the specimen of at least 1-2 cm, which has been validated by means of the wave with ultrafast ultrasound. However, this sensor allows using 1000 times less energy than ultrasound, and has no detectable thermal effect with an accuracy of 0.1 degrees. Therefore, it allows a better control of the energy deposited on the specimen, which is advantageous in case of *in vivo* samples.

A third object of the invention is a method of measuring torsional ultrasonic signals in a specimen, for the purpose of deriving from same mechanical parameters of elasticity and anisotropy of said specimen. The method is characterized in that it comprises performing the following steps using the sensor described above:
a) Contacting at least one side of the ring sections of the receiver and a side of the emitter with a surface of the specimen and connecting the connections of the receiver to a measuring instrument.
b) Polarizing the piezoelectric transducer elements in the perpendicular direction to the axis of rotation of the sensor of the rings, in the tangential direction to the ring defined by the base.
c) Emitting an electrical excitation signal that rotates the sensor to induce a torsional wave that goes through the specimen.
d) Receiving, in each of the piezoelectric transducer elements of the sensor, one or more electrical signals that are distorted after having gone through the specimen.
e) Prior to the acquisition, optionally, the signal of each of the piezoelectric transducer elements can be pre-amplified by means of a pre-amplifier.
f) Acquiring and storing, by means of a measuring instrument connected to the sensor, the excitation signal and the one or more distorted signals.
g) Optionally repeating steps a)-f) a plurality of iterations to refine the measurement (in order to have several measurements and averaging to improve the reliability of the measurement).

A plurality of distorted signals are thereby obtained for each of the channels (piezoelectric transducer elements) of the sensor, which obtain information dependent of the orientation of the microstructure of the specimen.

Once the measurements are obtained with the above method, said signals must be post-processed. For example, said post-processing comprises identifying the signals measured with mechanical models and using the inverse problem formalism, or other techniques known in practice, to characterize mechanical parameters (elasticity, stiffness, etc.). The present sensor allows discriminating said parameters by orientation, such that measurements of anisotropy can be obtained from the model-derived parameters.

In a preferred embodiment of the above method, the electrical signal used as excitation is an oscillating signal, more preferably a sinusoidal signal and even more preferably, a sine signal. In even more preferred embodiments, the excitation signal, generated by the generation means for generating an excitation signal, is sinusoidal and has a frequency between 100 Hz and 10 KHz, with an amplitude in the range of 1× **10⁻⁵** to 1× **10⁻²** radians, in a train of between 1 and 10 pulses; and wherein said signal can optionally be repeated to average the measurement, using a repetition period of between 1 and 50 ms.

A fourth object of the invention is the preferred use of the receiver and/or of the sensor described above to measure mechanical parameters, such as certain elastic properties (shear modulus, etc.) and/or anisotropy of anisotropic biological structures. More preferably, it is used to study anisotropy of the skin or other soft tissue. By means of the ultrasonic signals measured with the sensor, identifications can be mode with mechanical models and mechanical parameters obtained. Subsequently, given that the sensor allows distinguishing the measurements by orientations, it is possible to derive a measurement of anisotropy of the mechanical parameters obtained.

### DESCRIPTION OF THE DRAWINGS

To complement the description of the invention, a set of figures is attached as an integral part of the description which illustrate several preferred implementations of the invention. However, such implementations must not be interpreted as being restrictive of the scope of the invention, but rather only as different examples of how it can be carried out.
Figure 1 shows a view lateral of the receiver of the sensor of the invention.
Figure 2 shows an overhead view of the receiver of the sensor of the invention, comprising three ring sections.
Figure 3 represents the emitter and the securing parts thereof included in the sensor of the invention.
Figure 4 corresponds to the assembly of the receiver of Figures 1 and 2, and the emitter and the securing parts of the emitter shown in Figure 3.
Figure 5 shows a graph representing the values of the anisotropy ratio vs. the mean wave velocity (Cs, represented in m/s) values, obtained for a use example of the invention, in a preferred embodiment thereof. Said values are identified as "HX-Y", wherein H represents a healthy individual, X is the number of the individual, and Y is the number of the sample obtained from same. Similarly, pathological individuals are identified as "PX", wherein X is the number of the individual.
Figure 6 shows a graph representing the values of the amplitude of the shear wave velocity vs. the wave velocity (Cs, represented in m/s) values evaluated at the central frequency for all the vertical measurements, obtained for a use example of the invention, in a preferred embodiment thereof. Said values are identified as "HX-Y", wherein H represents a healthy individual, X is the number of the individual, and Y is the number of the sample obtained from same. Similarly, pathological individuals are identified as "PX", wherein X is the number of the individual.
Figure 7 shows a graph representing the values of the amplitude of the shear wave velocity vs. the wave velocity (Cs, represented in m/s) values evaluated at the central frequency for all the horizontal measurements, obtained for a use example of the invention, in a preferred embodiment thereof. Said values are identified as "HX-Y", wherein H represents a healthy individual, X is the number of the individual, and Y is the number of the sample obtained from same. Similarly, pathological individuals are identified as "PX", wherein X is the number of the individual.

**Reference numbers used in the drawings**

| | |
|---|---|
| (1) | Receiver |
| (2) | Casing |
| (3) | Emitter |
| (3') | Securing part(s) of the emitting device |
| (4) | Ring sections |
| (5) | Base of the ring |
| (6) | Transducer elements |
| (7) | Axis of rotation of the sensor |
| (8) | Direction of polarization tangential to the circumference defined by the rings |
| (9) | Support platform for the piezoelectric transducer elements |
| (10) | Side securing posts |
| (11) | Slots for guiding the connections of the receiver |
| (12) | Housing space (gap) for the emitter |
| (13) | Slots for guiding the connections of the emitter |
| (14) | Attenuators (elastomeric) |

### DETAILED DESCRIPTION OF THE INVENTION

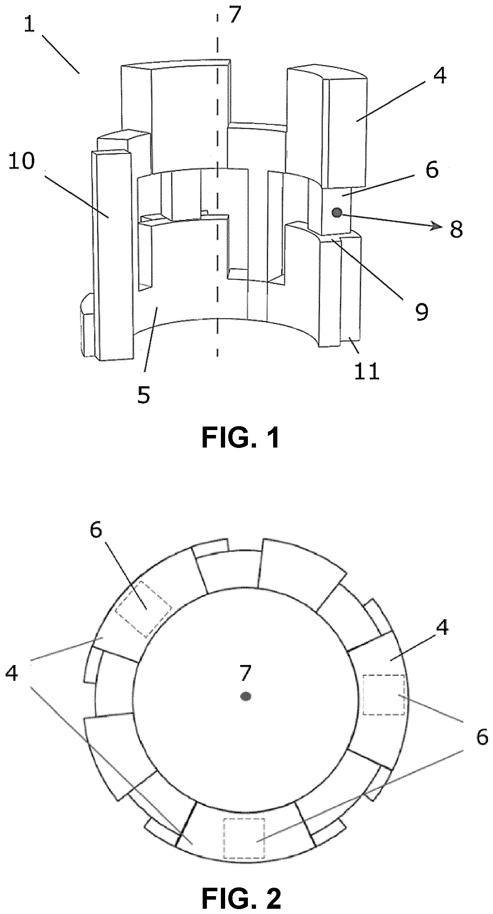

A detailed description of the invention in reference to various preferred embodiments thereof, based on Figures 1-4 herein, is provided below.

### Definitions

- Specimen or sample refers to the material, preferably a soft tissue, through which the waves emitted by the sensor are propagated so as to analyze the microstructure (elastic parameters, anisotropy, etc.) thereof.
- Excitation signal generator is understood to mean a device capable of generating waves or electrical signals, with said electrical signal being of an electric magnitude that varies over time (not necessarily periodically) and which can adopt various wave forms (sinusoidal, triangular, sawtooth, etc.). An example of a generator could be an oscilloscope.
- Biocompatible material is a material that does not degrade the measured biological specimen. In particular, the biocompatible materials used in this invention must preferably be sterilizable and compatible with direct contact (brief or prolonged) with the skin. An example of this material is the polylactic acid (PLA).
- Contact side refers to the part or surface of the sensor located in the distal or anterior part thereof (in particular, the upper part of the receiving ring) that comes into contact with the specimen in which the wave is applied. Preferably, the surface of said contact element will be substantially planar to allow a transmission suitable (without excessive attenuation) of the wave.
- The terms "upper side" and/or "lower side" of the ring of the sensor refer to the orientation thereof as reflected in the figures attached to the description. Similarly, "inner side" of the ring is understood to mean that side which is closest to the axis of the sensor.
- The term "conductive material" or "non-conductive material" refers to the electrical conductivity thereof.
- The term "acoustically conductive material" efficiently allows the transmission of ultrasonic waves. In the scope of this invention, some examples of materials of this type are considered to be certain photopolymers such as biocompatible PolyJet MED610, stereolithography (SLA) resin or selective laser sintering (SLS) resin, among others with similar acoustic properties (acoustic impedance, attenuation, etc.).
- The acoustic connection between two materials will be understood to mean that connection which allows the propagation of mechanical, preferably ultrasonic, waves between such materials.
- The term "receiving ring" refers to a three-dimensional ring comprising ultrasonic wave receivers. Said ring is not complete, but rather is a sectioned ring exhibiting gaps (slots) and in which a plurality of sections are therefore missing along the contour thereof. The sections of the ring are not necessarily equally spaced apart nor do they necessarily have the same geometric dimensions.
- The amplitude of the signals measured with the sensor are defined in radians, since the output signal that is measured in the transducer elements (for example, piezoelectric elements) is an electrical signal (for example, measured as a voltage in volt units). However, said signal is a result of a mechanical displacement which, for the particular case of torsional waves, is a rotation, so it can be measured in radians as the angular displacement induced by said rotation. As a result, in the scope of the present invention the amplitude of the signal is defined as an angle.
- In the context of the present invention, the concept of "ultrasound" will be understood in the broad sense, where it is equivalent to a mechanical wave of any nature, including both primary (purely acoustic) P-waves and secondary S-waves. Furthermore, "ultrasound" includes mechanical waves without being restricted to the spectrum of standard frequencies (greater than 20 KHz), but rather it could be generalized to another range of frequencies, including audible frequencies.
- In the scope of the invention, similarly the expression "substantially" will be understood to mean identical or comprised in the margin of variation of ±5%.

The device object of the invention (hereinafter "sensor") is a sensor based on a technology that generates and detects torsional waves in tissue. This sensor can generate and measure in skin tissue and is capable of interrogating with precision the mechanical functionality of the ageing of the skin, being propagated without some commercial elastography limitations in small organs. Advantageously, this sensor prevents the measurement of spurious compressional wave components which mask shear waves in soft tissue and improves current shear wave propagation models.

The sensor is adapted to emitting shear or torsional waves (ignoring the contribution of spurious longitudinal waves) so as to convert them into electrical signals. Furthermore, the said sensor is also adapted to receive torsional waves after going through a specimen. The sensor can thereby quantify elastic parameters, for example, the shear modulus of the specimen (preferably an anisotropic external structure, such as the skin), in at least three different directions.

Namely, the sensor object of this invention comprises three main elements: a receiver (1), a casing (2) which houses the receiver (1); and an emitter (3) attached to one or more securing parts (3') of said emitter (3), which are also housed in the casing (2).

Each of these elements, as well as different particular embodiments thereof are described below in greater detail.

### Receiver (1) of the sensor

The receiver of the sensor (Figure 1) exhibits an annular (for example, circular) geometry with two different partes: an upper part formed by a plurality of ring sections (4); and a lower part or base (5), also preferably having an annular geometry but designed as a complete ring (a single part, without sections). The base (5) must be hollow so as to enable inserting the emitter (3) therein. At least one surface of each of the ring sections (4) (the upper sides thereof) are in contact with the sample to be measured (tissue) during the measurement.

In a particular embodiment, the annular base (5) and the ring sections (4) are manufactured with photopolymers with at least the following characteristics:
- S-wave velocities compatible with soft tissue and respecting the energy safety limits applied to same to prevent damage. In this regard the safety standard recommended by the United States Food and Drug Administration (FDA), which indicates acceptable ultrasound ranges (about 720 mW/cm², mechanical index not to exceed 1.9 for acoustic power, thermal index below 1°C), should be mentioned.
- Acoustic impedance compatible with soft tissue.
- Limited acoustic attenuation.
- Sterilizable.
- Showing biocompatibility on contact with the skin (this would be sufficient since it is not necessary to ingest for the application of skin elasticity measurement).

Examples of photopolymers that can be used are biocompatible PolyJet MED610, stereolithography or SLA resin, selective laser sintering or SLS resin.

The sensor also comprises a plurality of transducer elements (6) which are arranged around the axis of the receiver (1) along the circumference defined by the annular base (5), with a polarization (8) tangential to the circumference defined by the base (5). The transducer elements (6) are attached both to the base (5) and to the ring sections (4). Similarly, the receiver (1) comprises support platforms (9) of the transducer elements (6) and side securing posts (10) and slots (11) for guiding the connections of the receiver.

In a preferred embodiment, the transducer elements (6) are piezoelectric elements, although these transducers can be capacitive transducers or of other types that allow efficiently converting torsional waves into an electrical signal.

In a particular configuration (Figure 2, top view of the receiver), the axis (7) of the sensor represents both the axis of rotation of the emitter and the axis of rotation of the receiver (i.e., both of the annular base (5) and of the ring comprising the plurality of ring sections (4). In this particular arrangement of the sensor, both axes of rotation thereby coincide and define the axis (7) of the sensor.

The upper sides of the ring sections (4) are the contact elements that will transmit an oscillating rotation (torsional wave) to the specimen. Subsequently, by means of a plurality of independent piezoelectric transducer elements (6) (preferably three, one per axis to be measured), preferably manufactured with lead zirconate titanate ceramic (hereinafter abbreviated as PZT), the signal coming from each of the measured directions will be collected once it goes through the specimen.

In a particular embodiment, the transducer elements (6) are manufactured from NCE51, a piezoelectric Noliac material having good acoustic properties for applications requiring a high coupling factor and high charge sensitivity.

In another particular embodiment, the same number of platforms (9) as transducer elements (6) are arranged.

### Emitter (3) of the sensor

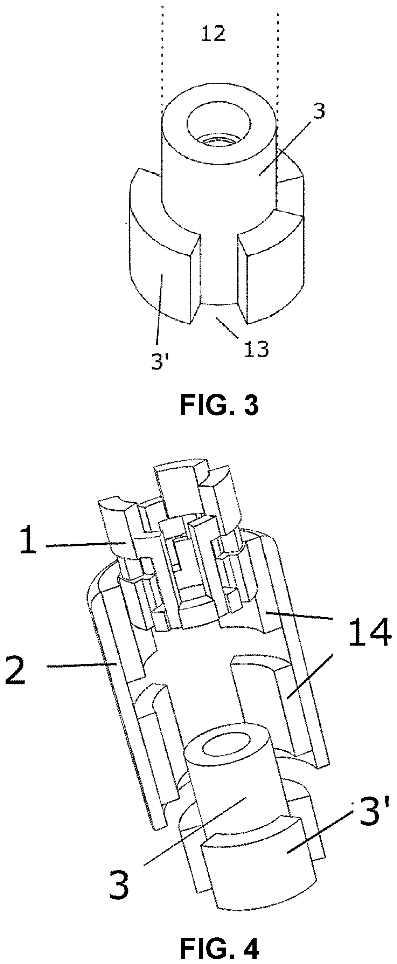

The emitter (3) of the invention (Figure 3) is a torsional ultrasonic wave emitting device preferably comprising an electrical excitation signal generator, attached to an electromechanical actuator which is in turn attached to the upper sides of the ring sections (4) which act as the contact element for contacting the specimen. When the electromechanical actuator receives electrical signals, it induces a rotational movement in the ring sections (4) and these sections, upon coming into contact with the sample, induce a torsional wave that goes through the specimen.

Additionally, the transductor comprises one or more parts (3') that allow securing the emitter (3). The securing parts (3') are adapted to house the emitter, such that it is aligned with the axis (7) of the sensor and arranged substantially centered with respect to the center of the annular base (5). Advantageously, this alignment mitigates measurement errors since the noise in the measurement obtained with the transductor is proportional to the error associated with the misalignment between the axis of the annular base (5) and the axis of the securing element (3') of the emitter (3). Figure 3 also illustrates the housing space (12), delimited by the securing elements (3'), in which the ultrasonic emitter (3) is introduced in a tight manner, as well as the slots (13) for guiding the connections of the emitter (3). At least one surface of said emitter must have access to a surface of the sample to be measured.

In a preferred embodiment, the complete ring constituting the base (5) and the ring sections (4) have the same thickness.

### Casing (2) of the sensor

Figure 4 shows the assembly of the complete sensor, including both the receiver (1) of Figures 1 and 2 and the emitter (3) of Figure 3.

The casing (2) comprises a considerably cylindrical element, which houses therein the emitter (3), the receiver (1), and the securing element (3') of the emitting device, and comprises attenuators (14), manufactured in an elastic material (for example, an elastomeric material can be used), located in the inner part thereof. Both the emitter (3) and the receiver (1) are thereby separated from one another. The cylindrical element is preferably manufactured in a material with the following characteristics:
- S-wave velocities compatible with soft tissue and respecting the energy safety limits applied to same to prevent damage.
- Acoustic impedance compatible with soft tissue.
- Limited acoustic attenuation.
- Sterilizable.
- Showing biocompatibility on contact with skin.
- Acoustic impedance thereof must be substantially different from the impedance of the material of the annular base (5)

In that sense, the material used for said cylindrical element, or for the casing itself, is preferably polylactic acid (hereinafter referred to as PLA).

The casing (2) prevents the masking of S-waves due to the P-waves. Preferably, the casing (2) is manufactured by means of 3D printing so that the measurements are suited for housing the rest of the elements of the device in a tight manner, maintaining the alignment and centering between emitter and receiver.

### Other characteristics of the sensor

In another particular embodiment, the sensor further comprises an aluminum sheet arranged forming an overlay on the electromechanical actuator and the conductive elements thereof to separate the connections of the emitter (3) and receiver (1).

Preferably, the attachment of the electromechanical actuator (which induces torsion in the specimen) and the aluminum overlay thereof is carried out with conductive silver resin. In this way, the isolating effect of the Faraday cage is even further enhanced and the possible noise that may arise if said aluminum cage is accidentally perforated is minimized.

### PREFERRED EMBODIMENT OF THE INVENTION

The embodiment of the sensor object of the invention is described below in a non-limiting manner.
- The receiver (1) consists of a sectioned ring comprising:
   a) An annular base (5) (complete ring) manufactured in biocompatible PolyJet MED610, a biocompatible photopolymer, and having the following dimensions: inner diameter of 9 mm and outer diameter of 13 mm. It has a series of slots (11) around the support platforms (9) located between posts (10) which will guide the connections (wiring) of said receiver.
   b) At least three ring sections (4) manufactured in the same plastic material and with the same dimensions as the annular base (5) and arranged on said ring, as observed in Figure 2. Each of the ring sections (4) is arranged substantially parallel to each of the support platforms (9), being supported by the posts (10), to which they are integrally adhered.
   c) A conductive overlay arranged in the platforms (9) attached to each ring section (4) of the receiver (1) and on the inner side of each ring section (4), said overlay functioning as connections or electrodes. Each electrode further comprises a wired connection which will be guided to an oscilloscope or other measuring instrument through the slots (11) for guiding connections. Since the signal that is obtained is typically in the order or microvolts, it should first be pre-amplified.
   d) Three transducer elements (6) (as many piezoelectric elements as there are sections are arranged) manufactured from piezoelectric ceramic PZT-4 (PZT-5 could also be used), with dimensions of 1.5 × 1.5 × 2.5 mm, each of them fixed to one of the ring sections (4). Said transducer elements (6) are polarized in the circumferential direction, parallel to the rings, whereas the electrodes are located in the attachment between the transducer elements (6) and the inner side of each of the ring sections (4). For the movement to be torsional, it is essential for both the polarization and the orientation of the polarization direction to be in accordance with the circumference defined by the rings. The attachment of the transducer elements (6) and of the wiring to the electrodes is carried out with conductive silver resin.
- An ultrasonic emitter (3) integrally attached to a securing element (3'). The ultrasonic emitter (3) used in this case is an electromechanical emitter. The emitter is housed in the housing space (12) and has a contact surface where it can access the specimen. The securing element (3') is adapted to house the ultrasonic emitter in a tight manner, according to the geometry and dimensions thereof. The securing element (3') is coupled to the attenuators (14), preferably one or more layers of elastomer to dampen cross-talk or mechanical noise. Both the emitter and the receiver (1) are thereby aligned with respect to the axis (7) of the sensor, said axis being defined by the perpendicular direction to the circle delimited by the annular base (5) and containing the center thereof. In that sense, the securing element (3') of the emitting device provides structural stability to the assembly of the sensor and comprises several slots (13) for guiding the cables coming from said emitter.

In this embodiment, the complete ring constituting the base (5) and the ring sections (4) have the same thickness.

The number of platforms (9) used is equal to the number of transducer elements (6).

As an alternative to PZT-4 or PZT-5, the transducer elements (6) are manufactured from NCE51, a piezoelectric Noliac material having good acoustic properties for applications requiring a high coupling factor and high charge sensitivity.

The sensor further comprises an aluminum sheet arranged forming an overlay on the electromechanical actuator and the conductive elements thereof. The connections of the emitter (3) and receiver (1) are thereby separated from one another, thereby acting as a Faraday cage to prevent the electrical noise (cross-talk). It should be noted that cross-talk arises given that the actuator of the emitter (3) is subjected to a voltage during the measurement, inducing a magnetic field that could affect the ultrasonic receiver (1). To minimize that cross-talk, the Faraday cage is implemented, preventing emitter and receiver (1) resonance. For torsional waves, said frequency can be determined by means of a conventional computing program that allows the simulation of the sensor by finite elements. The excitation can thereby be adapted depending on the resonance frequency of the sensor.

The attachment of the electromechanical actuator (which induces torsion in the specimen) and the aluminum overlay thereof is carried out with conductive silver resin.

In preferred embodiments of the invention, the contact element for contacting the specimen (the upper side of the ring sections (4)) comprises biocompatible material.

The design, geometry, and dimensions of the preceding embodiment have been optimized by means of a finite element computing program for the purpose of maximizing the signal-to-noise ratio (abbreviated as SNR) of the measurements of the distorted signal obtained by the sensor after going the specimen. With this configuration, the wave transmitted by the sensor is a torsional wave with a high signal-to-noise ratio (e.g., 5-10 dB).

The functionality of the device is assured by arranging a contact element (a surface) of the emitter (3) in the same plano as the upper side of the ring sections (4) of the receiver (1). This relative positioning is achieved by means of the placement of the attenuators (14) in the casing (2), which are snapped onto same. Similarly, the securing element (3') of the emitter (3) allows positioning the surface of the emitter (3) which is in contact with the plane where the specimen is located so that the axis of rotation thereof coincides with the axis (7) of the sensor.

Hereinafter, it will be assumed that the method is used for measuring the elasticity of an anisotropic structure, for example, the skin or another biological tissue. However, the sensor of the invention is not limited to this use. In this case, the sensor described above emits torsional waves and operates following the method described below for measuring the elasticity and/or anisotropy in a sample (in this case skin):
a) Contacting the side outer of the sensor (comprising both the upper side of the ring sections (4) and the surface of the ultrasonic emitter) with the surface of the specimen (skin). A planar surface of the skin is chosen, and a moderate pressure, preferably between 50-200 g/cm² (4903.325-19613.3 Pa) will be applied, and it is not necessary to apply coupling material between the specimen and the outer side of the sensor. The orientation of the sensor must be that of the preferred direction of the fibers in the skin in order to control the orientation of the measured anisotropy. To that end, some type of visual mark can be made in the casing (2) which serves to suitably orient the sensor. Typically anatomists know the preferred directions and, for certain tissue, it is even possible to recognize or intuit them with the naked eye or by observation with a suitable instrument (microscope, etc.). The connections of the receiver are connected to a measuring instrument (for example, an oscilloscope or an A/D converter for the output of the signals).
b) Polarizing the piezoelectric transducer elements in the perpendicular direction to the axis of rotation of the sensor, in the tangential direction to the ring defined by the base.
c) Emitting an excitation signal, which is preferably sinusoidal and has a frequency between 100 Hz and 10 KHz, with an amplitude in the range of 1×10⁻⁵ a 1×10⁻² radians, in a train of between 1 and 10 pulses. Similarly, said signal can optionally be repeated (for example, between 10 and 100 times) to average the measurement and improve the reliability thereof, using a repetition period of between 1 and 50 ms.
d) Propagating the signal in the specimen.
e) Receiving the signal distorted by the specimen in the plurality of transducer elements (6). The signal distorted by the sample is received in each of the transducer elements (6) through the connections (wiring) constituting a channel, which is independent of the rest of the channels. Each channel records the distorted signal in one orientation, which allows analyzing the anisotropy of the mechanical parameter detected. Each channel is measured by means of the measuring instrument.
f) Repeating the above steps as many times as measurements are required.
g) Performing a post-processing of the distorted signal to extract one or more quantitative mechanical parameters of elasticity and/or anisotropy. The principle for mechanically characterizing the microstructure of a sample is based on the fact that a physical parameter is propagated in the form of a wave through the specimen, which distorts the wave, said wave being received and measured on an accessible surface of said specimen. The mechanical parameters responsible for the modification of the wave (for example, distortion of its shape or variation of the amplitude) can be deduced by applying the model-based inverse problem. Optionally, the signal or signals measured with the sensor can be amplified and/or filtered prior to the acquisition and storage thereof. Therefore, the post-processing may comprise both processing by hardware (by means of signal filters and amplifiers) and software (acquisition, digitalization, noise reduction, etc.) for conditioning the acquired signal.

Based on the measurements obtained with the sensor, several biomarkers, such as shear modulus and shear viscosity, are obtained. Said parameters are obtained for each channel (angle/orientation), i.e., for each of the transducer elements (6) located in the different ring sections (4). Another biomarker that can be measured is the anisotropy, which is defined as the ratio between the value of the biomarkers in the longitudinal direction of the fiber (defined as 0°) and the value thereof in the transverse direction (90°). Optionally, one or more measurements can be taken for redundancy purposes (for example, at 45° and/or 225°), which can also be useful if more complex models of the sample are considered. Other angular combinations for arranging the transducer elements (6) may also be valid.

In that sense, with more complex models, more parameters of elasticity and anisotropy could be extracted.

A preferred use of the device and method described is the study of the elasticity and anisotropy of the skin under *in vivo* conditions. However, it could be used in an equivalent manner in any anisotropic material and not necessarily *in vivo.*

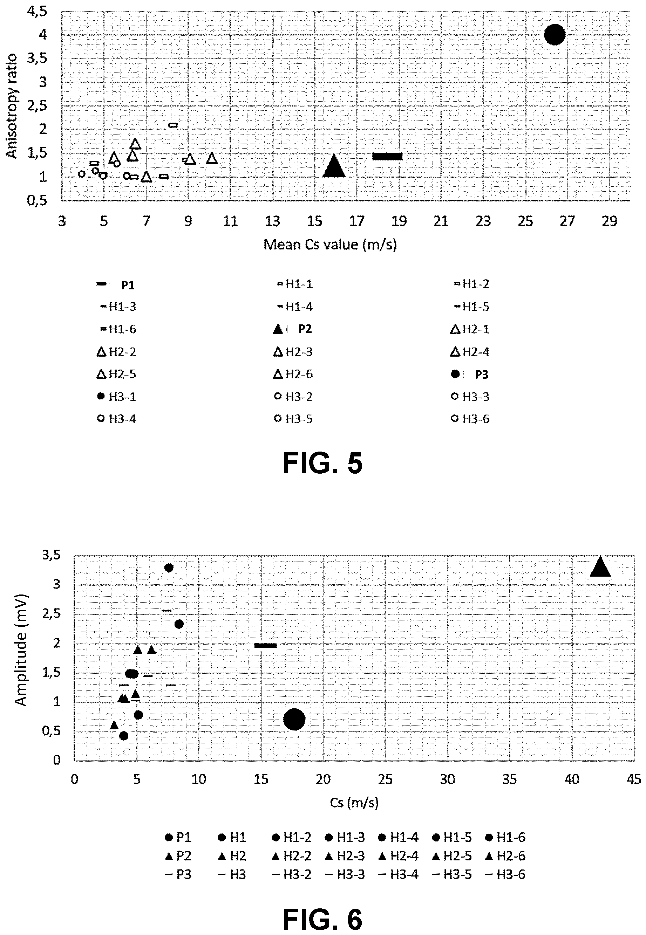

By way of example of preferred use of the invention, Figure 5-7 show a series of graphs which represent different values obtained for a group of both healthy and pathological individuals by means of the ultrasonic sensor of the invention. More specifically, pathological individuals exhibited preliminary diagnoses of different types of skin cancer (basal cell carcinoma, BCC, squamous cell carcinoma, SCC, and intra-oral basal cell carcinoma). The results obtained in these patients were compared with the results obtained in healthy individuals.

To obtain the results, the pathological tissue (lesion) was first examined and the healthy tissue (symmetrical area) was then also analyzed according to the site in the body and the location of the lesion, in order to compare the results acquired. The same test method with the same sensor was also carried out in healthy volunteers in order to provide greater understanding of the results.

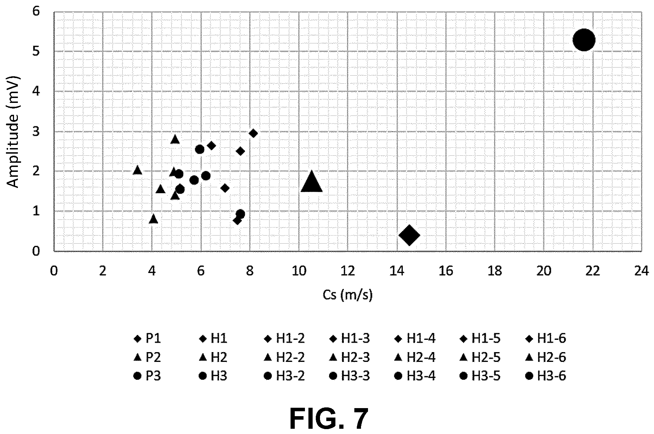

Namely, Figure 5 represents the values of the anisotropy ratio vs. the mean wave velocity (Cs) values. Similarly, Figure 6 represents the values of the amplitude of the shear wave velocity vs. the wave velocity (Cs) values evaluated at the central frequency for all the vertical measurements, and Figure 7 represents the values of the amplitude of the shear wave velocity vs. the wave velocity (Cs) values evaluated at the central frequency for all the horizontal measurements. Said values are identified as "HX-Y", wherein H represents a healthy individual, X is the number of the individual, and Y is the number of the sample obtained from same. Similarly, pathological individuals are identified as "PX", wherein X is the number of the individual.

As can be seen in the graphs shown, pathological individuals can be clearly distinguished from healthy individuals for the three pathologies studies.

Therefore, it can be seen that the objects (device and method) of the invention set forth previously, which are inferred from the above description, are achieved in an efficiently manner. Similarly, it is also possible to make modifications when carrying out the above method with respect to the preferred embodiments set forth without departing from the spirit and the scope of the invention, where it can be understood that the subject matter contained in the above description and shown in the attached drawings is interpreted as being illustrative and non-limiting.

## Claims

1. An ultrasonic receiver (1) adapted to receive torsional waves coming from a specimen, **characterized in that** said receiver (1) comprises:
- a receiving ring manufactured from an acoustically conductive material, wherein said receiving ring comprises a plurality of ring sections (4) separated from one another and arranged around an axis of rotation of the receiver (1) along a circumference;
- a plurality of transducer elements (6), each of them acoustically connected to one of the ring sections (4); and
- supporting means that support the receiving ring.

2. The ultrasonic receiver (1) according to the preceding claim, wherein the transducer elements (6) are piezoelectric or capacitive elements.

3. The ultrasonic receiver (1) according to any of the preceding claims, wherein each transducer element (6) further comprises one or more connections suited for receiving electrical signals and for connection thereof to a measuring instrument, with said connection being direct or after amplification of the electrical signal received in the connections with an amplifier.

4. The ultrasonic receiver (1) according to any of the preceding claims, wherein the supporting means comprise an annular base (5) on which the transducer elements (6) arranged between said base (5) and the receiving ring are distributed; and wherein the base (5) further comprises a plurality of slots (11) for guiding the connections of said receiver (1).

5. The receiver (1) according to any of the preceding claims, wherein the base (5) comprises non-electrically conductive material, and wherein at least one side of the ring sections (4) is manufactured from biocompatible material and is suited for receiving torsional waves coming from a specimen when in contact with said specimen.

6. An ultrasonic sensor for emitting and receiving torsional waves through a specimen, **characterized in that** it comprises:
- an ultrasonic receiver (1) according to any of the preceding claims;
- generation means for generating one or more electrical excitation signals;
- an ultrasonic emitter (3), attached to the receiver (1), comprising an electromechanical actuator connected by means of one or more connections to the generation means for generating an excitation signal, wherein said actuator is adapted to induce the rotation of the ring sections (4) of the receiver (1) around an axis of rotation of the electromechanical actuator of the emitter (3) upon receiving one or more electrical excitation signals; and wherein the emitter (3) comprises a side adapted to be arranged in contact with the specimen;
- acquisition and storage means for acquiring and storing the signals received in the connections of the receiver (1) and of the emitter (3) independently.

7. The ultrasonic sensor according to the preceding claim, wherein the axis of rotation of the receiver (1) and the axis of rotation of the electromechanical actuator of the emitter (3) coincide substantially with the axis (7) of the sensor.

8. The ultrasonic sensor according to any of claims 6-7, further comprising a casing (2) which houses the emitter (3) and the receiver (1), substantially maintaining the alignment between their axes of rotation; wherein said casing (2) further comprises slots (13) for guiding connections of the emitter (3).

9. The ultrasonic sensor according to any of claims 6-8, further comprising:
- one or more securing parts (3') for securing the emitter (3) manufactured from non-electrically conductive material and delimiting a housing space (12) in which the emitter (3) is inserted and a plurality of slots (13) for guiding connections of the emitter (3); and
- optionally, alignment means adapted to position the one or more securing parts (3') to align the axes of rotation of the emitter (3) and of the receiver (1).

10. The ultrasonic sensor according to claims 8-9, further comprising attenuators (14) arranged between the casing (2) and the emitter (3), and between the casing (2) and the receiver (1), with said attenuators (14) being adapted to dissipate the mechanical wave which is propagated through the casing (2).

11. The ultrasonic sensor according to any of claims 6-10, wherein the supporting means comprise a plurality of support platforms (9) for the transducer elements (6), with at least two side securing posts (10) emerging from each of said platforms (9); and wherein each post (10) further comprises a plurality of slots (11) for guiding the connections of the receiver (1).

12. The ultrasonic sensor according to preceding claim, **characterized in that** the electrodes of each transducer element (6) have connections independent from one another, said connections being suited for measuring therein an electrical signal obtained with the sensor by means of a measuring instrument; and with said electrodes being connected to a side of the receiving ring with silver conductive resin.

13. The ultrasonic sensor according to any of claims 6-12, further comprising a Faraday cage covering the electromechanical actuator of the emitter (3), to eliminate electrical noise from the resonance of the emitter (3) and of the receiver (1).

14. The ultrasonic sensor according to any of claims 6-13, comprising three ring sections (4) and three transducer elements (6) oriented respectively with respect to the axis (7) of the sensor at 0°, 90°, and optionally one of the following angles, 45° or 225°.

15. A method for measuring torsional waves in a specimen, **characterized in that** it comprises performing the following steps using the sensor according to any of claims 6-14:
a) contacting a side of the ring sections (4) of the receiver and a side of the emitter (1) with a surface of the specimen and connecting the connections of the receiver to a measuring instrument;
b) polarizing the transducer elements (6) in the perpendicular direction to the axis (7) of rotation of the sensor, in the tangential direction (8) to the ring defined by the base (5);
c) emitting an electrical excitation signal to rotate the sensor and induce a torsional wave that goes through the specimen;
d) receiving, in each of the transducer elements (6), one or more electrical signals that are distorted after having gone through the specimen;
e) optionally amplifying the signal received in each of the transducer elements (6) by means of a pre-amplifier;
f) acquiring and storing, by means of a measuring instrument connected to the sensor or to the pre-amplifier, the excitation signal and the one or more distorted signals;
g) optionally repeating steps a)-f) a plurality of iterations and averaging the distorted signals acquired in each of the transducer elements (6).

16. The method according to the preceding claim, wherein the excitation signal is sinusoidal and has a frequency between 100 Hz and 10 KHz, with an amplitude between 1 ×10⁻⁵ and 1×10⁻² radians, in a train of between 1 and 10 pulses; and wherein said signal can optionally be repeated to average the measurement, using a repetition period of between 1 and 50 ms.

17. Use of the receiver (1) according to claims 1-5 and/or of the sensor according to claims 6-13 for measuring elastic properties and/or anisotropy of anisotropic biological structures.
